# EUROPEAN PATENT APPLICATION

(11) **EP 0 793 971 A1**
(43) Date of publication of application: **10.09.1997**
(21) Application number: 96830098.8
(22) Date of filing: 07.03.1996
(51) Int. Cl.: A61L 15/18, A61L 15/42

(54) **Absorbent articles comprising coagulant**

(71) Applicant: THE PROCTER & GAMBLE COMPANY, Cincinnati, Ohio 45202 (US)
(72) Inventor: Bewick-Sonntag, Christopher Philip, 65125 Pescara (IT); Marinelli, Luigi, 65129 Pescara (IT); Morena, Marco, 65016 Montesilvano sp.(PE) (IT); Veglio, Paolo, Pescara (IT)
(74) Representative: Hirsch, Uwe Thomas

(57) **Abstract**

The present invention relates to an absorbent article having a breathable backsheet and comprising a coagulant. The absorbent article exhibits reduced wet through onto the users garments.

## Description

### Field of the Invention

The present invention relates to thin absorbent articles having a breathable backsheet which exhibit reduced wet through onto the users garments.

### Background of the Invention

The primary consumer needs which underlie development in the absorbent article field, in particular catamenials is the provision of products providing both a high protection and comfort level.

One highly desirable means for improving the comfort of absorbent articles is the use of so called 'breathable backsheets'. Breathable backsheets may typically comprise either a microporous film or an apertured formed film preferably having directional fluid transfer as disclosed in for example US 4 591 523. Both types of breathable backsheets are vapour permeable, allowing gaseous exchange with the environment. This thereby allows for the evaporation of a portion of the fluid stored in the core and increases the circulation of air within the absorbent article. This is particularly beneficial as it reduces the sticky feeling experienced by many wearers during use, particularly over extended periods of time. Obviously the larger the apertures the greater the breathability of the backsheet.

However, the main drawback associated with the use of breathable backsheets in absorbent articles is the increased probability of leakage, commonly referred to as wet through, onto the users garment. Such leakage also increases proportionally to the aperture sizes of the breathable backsheet. Although the breathable backsheets in principle only allow the transfer of materials in the gaseous state, physical mechanisms such as extrusion, diffusion and capillary action may still occur and result in the transfer of the fluids from the absorbent core through the backsheet and onto the users garments. In particular, these mechanisms become more dominant if the product is utilised during physical exertion, for heavy discharge loads or over extended periods of time. Thus, whilst the incorporation of breathable backsheets in absorbent articles is highly desirable from a comfort standpoint, they result in an unacceptable level of failure with regard to protection, especially under stressed conditions.

The problem of wet through onto users garments due to the incorporation of such breathable backsheets in absorbent articles has been recognised in the art. However attempts to solve the problem have mainly resided in the use of multiple layer backsheets such as those illustrated in US 4 341 216. Similarly unpublished European patent application no. 94203228 discloses a breathable backsheet for disposable absorbent articles comprising an outer layer of a gas permeable, hydrophobic, polymeric fibrous fabric and an inner layer comprising an apertured formed film having directional fluid transport. Also unpublished European patent application no. 94203230 discloses breathable absorbent articles comprising a breathable backsheet consisting of at least two breathable layers which are unattached to one another over the core area.

None of the above solutions have satisfactorily addressed the problem of backsheet wet through. Furthermore, the above described solutions result in an increase of the overall thickness of the products due to the presence of an additional layer. Thus, these solutions are not applicable for incorporation within so-called thin products. However, thin products are highly desirable to the consumer particularly from a comfort standpoint. Moreover, the problem of wet through due to the presence of breathable backsheets is further accentuated when utilised within these thin type products.

As a result, there exists a need to provide an absorbent article which offers improved comfort by the employment of a breathable backsheet and having a reduced thickness which maintains the desired level of protection and exhibits reduced wet through.

It has now been found that breathable backsheets may be utilised in sanitary napkins having reduced thickness, thereby providing a product having both a high level of protection and comfort by the utilisation of a coagulant within the absorbent article.

The use of various compounds to coagulate human discharge contained in disposable articles has been described in the art, for example US 4 381 784 discloses the use of blood gelling agents in sanitary napkins. Similarly US 3 329 145 describes sanitary napkins comprising a fluid control element comprising a thin layer of fibrous material containing in admixture, pectin and an acid to coagulate proteinaceous material. Likewise, US 4 026 291 discloses the use of cationised cellulose fibres containing nitrogen in the absorbent core of compact articles for deodourising, sterilising and coagulating the fluids of the human body. The article also comprises an inner waterproofing member for preventing the fluid from permeating to the backside of the article. The use of carboxymethylalginate (CMA) and alginic acid salt incorporated into fibres in catamenial products for blood coagulation is discussed in US 5 080 657.

However, none of the above identified prior art relating to the use of blood coagulating agents in absorbent article recognises their application in combination with breathable backsheets to reduce wet through.

### Summary of the Invention

The present invention relates to an disposable absorbent article comprising, a liquid pervious topsheet, an absorbent core and a breathable backsheet, said core being intermediate said topsheet and said backsheet. The absorbent article comprises a coagulant.

### Detailed Description of the Invention

The present invention relates to absorbent disposable articles such as sanitary napkins, baby diapers, incontinence products and panty liners. Typically such products comprise a liquid pervious topsheet, a backsheet and an absorbent core intermediate said topsheet and said backsheet. An essential feature of the absorbent article of the present invention is the presence of a breathable backsheet which allows the circulation of air and vapour through it. It has now been found that such backsheets may be incorporated into absorbent articles without significant wet-through occurring onto the users garments by the incorporation of a coagulant in the absorbent article.

### Coagulant

Thus according to the present invention the absorbent article comprises as an essential feature a coagulant. The term coagulant as used herein refers to materials which exhibit the ability combine, aggregate, preferably irreversibly or increase the viscosity of semisolid particles to form a coagulum, clot or mass. In catamenial products this is principally the proteinaceous material present in menstrual blood i.e. blood cells and plasma proteins. Thus, in the present application the coagulant is a protein reactive chemical which reacts with the proteinaceous constituents of menstrual fluids to form a coagulum. In order to be effective the coagulant must act with the proteins contained in the menstrual fluids during the typical period for which the absorbent article is used and before the article is removed and if necessary replaced. This period will vary depending on the users habits, which depend on a number of variables such as the type of pad utilised, the rate of flow of the menstrual fluid and temperature. Typically, the coagulant should become effective i.e. initiate coagulation at least after 5 minutes, preferably after at least 2 minutes, most preferably immediately after coming into contact with the menstrual fluid.

In addition to protein coagulation a further advantage of the presence of the coagulant in the absorbent article is that the actual formation and presence of the coagulum also acts to hinder the movement of other non proteinaceous components of the discharge. Thus, in effect the coagulant hinders the movement of all of the components of the menstrual fluid and other fluids such as urine which may be present.

The coagulant agents for use in the absorbent articles of the present invention may be water soluble or water insoluble. Suitable coagulant agents for use herein include aluminum or chrom ammonium disulphate or aluminium or chrom potassium disulphate, iron salts, such as iron chloride, di-carboxylic anhydrides and polycations. Also suitable as coagulating agents are combinations of compounds such as in admixture, pectin and an acid selected from phosphotungstic, tannic and sulphosalicyclic acids. Also suitable are cationized cellulose fibres containing nitrogen and a compound mixture consisting of at least tannic acid, aluminum ammonium disulphate, ferric chloride, polyethylene oxide, polyacrylic acid soda and caboxymethyl cellulose. Combinations of carboxymethylalginate (CMA) and alginic acid salt may also be utilised herein. Preferably the coagulating agent is aluminium or chrom ammonium disulphate or aluminium or chrom potassium disulphate and mixtures thereof. Effective coagulants suitable for use herein may be determined using the Drip test described herein after.

The amount of coagulant for use herein will depend on the product type itself, the type of coagulant utilised and its location within the absorbent article. Typically however when utilised in a sanitary napkin, the coagulant can be used at a level of from 0.1 g to 1.5g, preferably from 0.2g to 1g, more preferably from 0.25g to 0.75g.

The coagulant may also be located at any position within the absorbent article. Similarly, the coagulant may also be dispersed or distributed uniformly throughout the absorbent article, dispersed at specific areas of the absorbent article, dispersed throughout a specific layer or dispersed at specific areas of a specific layer of the article. The coagulant thus maybe distributed within the absorbent core, the backsheet or any layer thereof or any combination of layers thereof. In embodiments according to the present invention wherein the absorbent core contains a plurality of layers the coagulant is preferably located in the layer of the core adjacent to the backsheet.

The coagulant may be distributed onto the absorbent article by any means known in the art. The coagulant is typically delivered and distributed on the absorbent article or respective layers thereof in the form of a granulate or fine powder. The coagulant may be combined or otherwise with a solvent to allow spraying, coating or painting of the coagulant at the desired location. Alternatively, the coagulant may be incorporated within a layer during the manufacture of the layer itself.

### Backsheet

The absorbent articles according to the present invention comprise as an essential component a breathable backsheet. The backsheet primarily prevents the extrudes absorbed and contained in the absorbent structure from wetting articles that contact the absorbent product such as underpants, pants, pyjamas and undergarments. In addition however, the breathable backsheet of the present invention permits the transfer of vapour and preferably both vapour and air through it and thus allows the circulation of air into and out of the backsheet.

According to the present invention the breathable backsheet comprises at least one gas permeable layer. Suitable gas permeable layers include 2 dimensional, planar micro and macro-porous films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2 dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the backsheet comprises a macroscopically expanded film or an apertured formed film, the backsheet will typically have an open area of more than 5%, preferably from 10% to 35% of the total backsheet surface area.

Suitable 2 dimensional planar layers of the backsheet may be made of any material known in the art, but are preferably manufactured from commonly available polymeric materials. Suitable materials are for example Goretex (TM) or Sympatex (TM) type materials well known in the art for there application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition, the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at their terminating ends. Preferably said protuberances are of a funnel shape, similar to those described in US 3, 929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 4 637 819 and US 4 591 523.

According to the present invention the backsheet may comprise in addition to said gas permeable layer additional backsheet layers. Said additional layers may be located on either side of said gas permeable layer of the backsheet. The additional layers may be of any material, such as a fibrous layer, preferably such that they do not reduce the gas permeability of the backsheet.

The backsheet typically extends across the whole of the absorbent structure and can extend into and form part of or all sideflaps, side wrapping elements or wings.

According to the present invention the absorbent article can be used beneficially in the context of sanitary napkins and panty liners. However, the absorbent articles of the present invention may also find utility as bandages and wound dressings and incontinence products. The disposable article may thus have all those features and parts which are typical for products in the context of their intended use. In addition to the components previously described they typically comprise a topsheet and absorbent core.

### Absorbent core

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent core may have any thickness depending on the end use envisioned. In a preferred embodiment of the present invention wherein the absorbent article is a sanitary napkin or a panty liner, the core may have a thickness of from 15mm to 1mm, preferably from 10mm to 1mm, most preferably from 7mm to 1mm.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bonds. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part off the primary or secondary fluid distribution layer are odor control agents. Active carbon coated with or in addition to other odor control agents, in particular suitable zeolite or clay materials, are optionally incorporated in the absorbent structure. These components can be incorporated in any desired form but often are included as discrete particles.

### The topsheet

The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as non woven fabrics, films or combinations of both. In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314 and US 4 591 523.

The topsheet typically extends across the whole of the absorbent structure and can extend into and form part of or all of the preferred sideflaps, side wrapping elements or wings.

### Examples:

Examples representative of the present invention are given below. Each example was derived from a sanitary napkins produced under the name of "Always Ultra Normal" available form Procter and Gamble GmbH, Schwalbach/Germany which had been manufactured according to normal manufacturing procedures and then modified as described below.

### Example 1: Reference

In this example the breathable backsheet is composed of a uni-directional (one way) conical apertured film (CPT) made of Low Density PE {supplied by Tredegar Film Products B.V. Holland under the manufacturing code X-1522} and positioned in contact with the absorbent core composed of a standard airlayed tissue and absorbent gelling material. The garment facing surface of the backsheet is composed of a nonwoven laminate {14MB/14SB manufactured by Corovin GmbH in Germany under the trade name MD 2005}. The nonwoven laminate is composed of 14 gm² spunbond and 14 gm² meltblown. No additional treatments or modifications have been applied.

### Example 2:

Is an identical structure to that of example 1 except that an additional layer has been inserted between the garment facing surface of the absorbent core tissue {supplied by Walkisoft Denmark (material code: Metmar Kotka)} and the wearer facing surface of the apertured (CPT) uni-directional film backsheet {supplied by Tredegar Film Products B.V. Holland under the manufacturing code X-1522}). This layer is a standard 18 gsm wet-laid tissue {supplied by San Marco s.r.l. Italy under the manufacturing name 'San Marco- NBC-1993'}) that has been treated with a solution maintained at pH ∼4 of Aluminum Potassium Disulphate (CAS 7784-24-9) to a dry basis weight add-on level of about 3 gm².

### Example 3:

Is an identical structure to that of example 1 except that the garment facing surface of the absorbent core tissue {supplied by Walkisoft Denmark (material code: Metmar Kotka)} has been treated with a solution maintained at pH ∼4 of Aluminum Potassium Disulphate (CAS 7784-24-9) to a dry basis weight add-on level of about 50 gm².

### Example 4:

Is an identical structure to that of example 2 except that the uni-directional (one way) conical apertured film (CPT) made of Low Density PE backsheet is replaced by a 2-dimensional planar perforated PE film backsheet (supplied by Pantex S.p.A. Sulmona/Italy manufactured under the manufacturing code PF10). As in example 2 the garment facing surface of the absorbent core tissue {supplied by Walkisoft Denmark (material code: Metmar Kotka)} has been treated with a solution maintained at pH ∼4 of Aluminum Potassium Disulphate (CAS 7784-24-9) to a dry basis weight add-on level of about 50 gm².

### Example 5: (reference)

Is an identical structure to that of example 1 except that the breathable backsheet composed of an apertured uni-directional film and non-woven layer have been replaced with a microapertured film {supplied by Exxon Chemical Company under the manufacturing code Exxaire XBF-102W}. No additional treatments have been applied.

### Example 6:

Is an identical structure to that of example 5 except that the garment facing surface of the absorbent core tissue {supplied by Walkisoft Denmark (material code: Metmar Kotka)} has been treated with a solution maintained at pH ∼4 of Aluminum Potassium Disulphate (CAS 7784-24-9) to a dry basis weight add-on level of about 50 gm².

### Drip Retention Test:

The Drip Retention test detailed is utilised to evaluate the resistance of a porous layer to transmission of vaginal or menstrual discharges. It can be used as a simple measure of the effectiveness of a particular coagulant or viscosity.

In this test two initially identical airlayed tissue samples, one of which is been post treated with a coagulation or viscosity altering agent, are compared. The test requires two identical samples (5 cm x 5 cm) of absorbent airlayed tissue sample {supplied by Walkisoft Denmark (material code: Metmar Kotka, 63 gsm)}. The lower surface of one of the samples has been subsequently coated with a 57% solution (maintained at pH ∼4) of Aluminium Potassium Sulphate (CAS 7784-24-9) at a temperature of 94 degree Centigrade and dried at about 80 degree Centigrade to a dry basis weight treatment-add-on-level of about 50 gsm.

Both samples of tissue are then fluted/folded and inserted into the 60 mm wide opening of a standard filter funnel (available from Bicasa S.p.A.Bernareggio/Italy under the code AX 3120) in a manner identical to that usually adopted to filter / separate a liquid/solid suspension. Positioned about 3cm above the funnel opening above the samples to be analysed is a liquid delivery system. The liquid used is a pure fresh solution of difibrinated sheep's blood (available from Unipath S.p.A (Garbagnate Milanese/Italy)-having a viscosity in the range of 7 to 8 (units cStK)} maintained at room temperature. Below the funnel a white piece of blotting paper is positioned to provide a clean surface to catch and display any blood that may drip from the funnel on loading.

The sheep blood is dripped onto the tissue sample within the funnel at a rate of 3 ml / minute and in a second experiment at a rate of 0.5 ml / minute {allows for slower coagulation or viscosity agents to be assessed} and continued until the moment a droplet appears below the funnel on the white sheet of paper. The volume of blood retained by the sample on appearance of the drip is recorded.

As the liquid is introduced, if no drop appears, the level of liquid in the filter paper rises and the introduction of the liquid should be stopped to ensure the liquid height does not exceed the height of the tissue sample in the funnel (typically at about 5 ml load).

The retention volume at drip or on termination of the test is recorded at each delivery rate. For the above described example the results are:
- Sample treated:: Vᵣₑₜₐᵢₙ > 5 ml at either 0.5 or 3 ml/min. loading rate
- Sample untreated:: Vᵣₑₜₐᵢₙ < 1.5 ml at 0.5 ml/min. loading rate
Vᵣₑₜₐᵢₙ < 1.0 ml at 3.0 ml/min. loading rate

A coagulation or viscosity altering agent is deemed effective if the tissue sample containing the agent exhibits a volume of retention at least 10 % higher than the untreated sample at either loading rate.

## Claims

1. A disposable absorbent article comprising, a liquid pervious topsheet, an absorbent core and a breathable backsheet, said core being intermediate said topsheet and said backsheet and said absorbent article comprising a coagulant.

2. A disposable article according to any one of the preceding claims, comprising from 0.1g to 1.5g of said coagulant.

3. A disposable article according to any one of the preceding claims, comprising from 0.2g to 1.0g of said coagulant.

4. A disposable article according to any one of the preceding claims, wherein said coagulant is selected from aluminium potassium disulphate, chrom potassium disulphate, aluminum ammonium disulphate, chrom ammonium disulphate and mixtures thereof.

5. A disposable article according to any one of the preceding claims, wherein said breathable backsheet comprises at least one layer selected from an apertured polymeric film or a 2 dimensional planar apertured film.

6. A disposable article according to claim 5, wherein said layer is a 2-dimensional planar apertured layer, wherein said apertures have an average diameter of from 150 micrometers to 5 micrometers.

7. A disposable absorbent article according to claim 5, wherein said layer is a 2 dimensional planar apertured layer, wherein said apertures have an average diameter of from 90 micrometers to 200 micrometers.

8. A disposable article according to Claim 5, wherein said layer is an apertured polymeric film, wherein said apertures are have on average a diameter of from 100 micrometers to 500 micrometers.

9. A disposable article according to any one of the previous claims, wherein said breathable backsheet comprises at least two layers, a first layer comprising an apertured layer and a second layer comprising a fibrous layer.

10. A disposable article according to any one of the preceding claims, wherein said article is a sanitary napkin or a panty liner.
